(19) Europäisches Patentamt
European Patent Office
Office européen des brevets

(11) **EP 2 146 681 B1**

(12) **EUROPEAN PATENT SPECIFICATION**

(45) Date of publication and mention
of the grant of the patent:
**07.06.2017 Bulletin 2017/23**

(21) Application number: **08747128.0**

(22) Date of filing: **30.04.2008**

(51) Int Cl.:
***A61K 8/11*** *(2006.01)*    ***A61Q 11/00*** *(2006.01)*

(86) International application number:
**PCT/US2008/061927**

(87) International publication number:
**WO 2008/147619 (04.12.2008 Gazette 2008/49)**

(54) **IMPERMEABLE CAPSULES**

UNDURCHLÄSSIGE KAPSELN

CAPSULES IMPERMÉABLES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.05.2007 US 751081**

(43) Date of publication of application:
**27.01.2010 Bulletin 2010/04**

(73) Proprietor: **Colgate-Palmolive Company New York, NY 10022 (US)**

(72) Inventors:
• **PILCH, Shira**
  **Highland Park, NJ 08904 (US)**

• **MASTERS, James, G.**
  **Ringoes, NJ 08551 (US)**

(74) Representative: **Wibbelmann, Jobst et al Wuesthoff & Wuesthoff Patentanwälte PartG mbB Schweigerstrasse 2 81541 München (DE)**

(56) References cited:
**EP-A- 0 180 287        EP-A- 0 332 175**
**EP-A- 0 622 408        WO-A-98/27151**
**WO-A-99/59535          WO-A-2004/110613**
**WO-A-2007/012981       DE-A1- 19 926 714**

**Description**

BACKGROUND OF THE INVENTION

Encapsulation technology is widely used in a number of application areas. For example, microencapsulation is used in formulations where it is necessary to separate two ingredients, for example by formulating one inside a protective wall in a capsule, until the ingredients are combined upon use. Capsules are also useful to provide various active ingredients in a form that is more readily formulated. Examples include flowable capsules that contain active ingredients that are oily, hydroscopic, reactive, and the like.

In the oral care area, many ingredients of the formulations can be provided in the form of capsules. Non-limiting examples include flavorings, colors, oxidizing agents, and active ingredients. In some areas, the use of encapsulation technology in oral care products, especially in dentifrice formulations, is limited due to poor impermeability of the capsules within the dentifrice matrix during processing and storage periods. That is to say, the capsules tend to be too readily attacked by aqueous conditions during processing and storage. This results in premature release of the microencapsulated ingredients.

Techniques for making capsules for dentifrice applications include spray drying, complex coacervation, and emulsion techniques. The methods involve the use of polymers to form capsules via precipitation, polymerization, or coalescent processes. After the capsules are formed and formulated into dentifrice formulations, the polymer molecules that form the capsule shells exhibit a swelling phenomenon in the presence of water. Many factors are known that can enhance the swelling effect, such as the presence of salts, variations in pH, and the presence of surfactants. As the polymer swells, a point is reached at which leakage of the capsules results, so that the active materials maintained in the core of the capsules are released.

Because many applications depend on slowing, hindering, or inhibiting premature release of active ingredients, it would be desirable to provide capsules, capsules, and processes for making them that would result in less permeable (more impermeable) capsule walls.

BRIEF SUMMARY OF THE INVENTION

The present invention provides a capsule according to claim 1, a composition according to claim 7, and a method according to claim 8. Preferred features are defined in the dependent claims.

By strategically selecting a second polymer with a higher Hildebrand solubility parameter, the capsules can be made more impermeable to water. Upon exposure to an aqueous medium, the at least one first polymer in the capsule wall begins to swell, and water begins to leak into the capsule. At the same time, the faster swelling second polymer is attracted to the entering water. As the second polymer absorbs water and swells, the mean free space in the shell wall will decrease and hence the tortuous pathway for materials crossing the boundary of the wall will be enhanced. As a result, leakage rates across the capsule wall decrease.

DETAILED DESCRIPTION OF THE INVENTION

As used throughout, ranges are used as a shorthand for describing each and every value that is within the range. Any value within the range can be selected as the terminus of the range.

In the present invention, the capsule comprises a core and a shell surrounding and enclosing the core. The shell contains a first polymer having a first Hildebrand solubility parameter and a second polymer having a Hildebrand solubility parameter higher than that of the first polymer. In capsules of the present invention, at least one of the second polymer is crosslinked and the second polymer is present in an amount that is less than the first polymer. The shell can be continuous or discontinuous.

The solubility parameter is given by Hildebrand units, or $\delta/MPa^{1/2}$. As is well known, these indicate the SI units for the Hildebrand solubility parameter. In various embodiments, the solubility parameter of the second polymer is at least 0.5 Hildebrand units greater than that of the first polymer. In other embodiments, the solubility parameter of the second polymer is at least 1 Hildebrand unit greater than that of the first polymer.

The second polymer, which is more swellable than the at least one first polymer, is present in a minor amount in the capsules. That is, on the basis of the total weight of the polymers in the capsules, the second polymer is less than 50% of the total weight. In various embodiments, the second polymer makes up about 0.01% to less than 50%, about 1 to about 40%, about 1 to about 30%, about 1 to about 20%, or about 1 to about 10% by weight of the total polymers in the capsules. The percentages are based on the total amount of first and second polymer in the capsules, whether the first and second polymers are both found in the shell wall, or whether the second polymer is found in the core. In a non-limiting example, the shell contains about 1 to 40 parts of the at least one second polymer per 100 parts of the at least one first polymer.

In other aspects, a method of decreasing the water permeability of a capsule shell is provided. The shell is made of at least one first polymer and the method involves forming the capsule from a mixture of the at least one first polymer and at least one second polymer.

The capsules may be formed by spray drying, complex coacervation, and/or by emulsion techniques.

Suitable compositions are also provided that contain the capsules in acceptable carrier systems. In particular, oral care compositions are provided that contain the capsules in an orally acceptable carrier.

The methods provide for the enhanced impermeability of the capsule by utilizing a polymer with a greater swelling rate than the polymers that form the shell. The second polymer is adjacent to or within the shell structure so that it can adhere to or entangle with the shell structure. As the shell is exposed to water, the at least one second polymer will compete with the at least one first polymer and absorb the entering water. Upon absorbing the water, the swelling polymer will hydrate and swell, which reduces the mean free space in the shell wall. As the polymer absorbs water and swells, the mean free space in the shell wall decreases. As a result, the tortuous pathway from materials crossing the boundary increases significantly. Because the path between the core and the outside of the capsule becomes more tortuous by virtue of the swelling of the second polymer, the rate of water making its way along the tortuous pathway into the core, and likewise the rate of active material making its way along the tortuous pathway through the wall to the outside of the capsule will be decreased. Macroscopically, this is observed in the form of a slower, delayed, or extended release of the active material from the capsule.

As noted, the second polymer (more highly swellable) can be incorporated into capsules as a component of the core constituents. As the shell swells and water leaks into the capsules, the second polymer molecule in the core is attracted to the entering water. The water causes the polymer to swell and to adhere to the inner surface of the shell. The second polymer is a part of the shell polymer mixture at a certain level, preferably at a minor level compared to the at least one first polymer. The second polymer can be physically mixed or entangled with the shell first polymers.

In all cases, the mean free space in the shell wall will decrease as a result of the swelling polymer. The tortuous pathway for materials crossing the boundary will increase significantly and consequently leakage rates across the shell wall will decrease without altering the shell wall thickness.

In the embodiments described herein, the second polymer (also called the "greater swelling" polymer) is characterized by being more swellable in water than the at least one first polymer. A measure of swellability in water is the solubility parameter. As developed below, the solubility parameter scales are set up so that, the closer a polymer is to the solubility parameter of the solvent water, the more it will swell in water. Water swelling often leads to dissolution; to prevent this, the second polymer can be provided in crosslinked form to keep it from dissolving in use. In various embodiments, the solubility parameter of the second polymer is closer to the parameter of the solvent water than the solubility parameter of the first polymer is. As a practical matter, water has about the highest solubility parameter of any solvent, which is related to its relatively high heat of vaporization. Accordingly, in various embodiments, the solubility parameter of the second polymer is characterized as being "higher" than that of the at least one first polymer. The second polymer thus swells more because its solubility parameter, being higher than that of the first polymer, is closer to the solubility parameter of the solvent water.

The second polymer swells to a greater extent as indicated by its higher (closer to water) solubility parameter. For convenience, this property will be referred to as "faster swelling" even though strictly speaking the solubility parameters is a predictor of the extent or equilibrium of swelling, not its rate. It is normally observed that polymers that swell to a greater extent by virtue of a solubility parameter close to that of water also swell at a faster rate, to provide the advantages described herein.

Hildebrand Solubility Parameter

The Hildebrand solubility parameter is a numerical value that indicates the relative solvency behavior of a specific solvent. It is derived from the cohesive energy density of the solvent, which is in turn derived from the heat of vaporization. The cohesive energy density SI is derived from

$$c = \frac{\Delta H - RT}{V_m} \qquad (1)$$

wherein c is cohesive energy density; $\Delta H$ is the heat of vaporization; R is the gas constant; T is the temperature; and $V_m$ is the molar volume. The Hildebrand Solubility Parameter is taken as the square root of the cohesive energy density according to

$$\delta = \sqrt{c} = \left[ \frac{\Delta H - RT}{V_m} \right]^{1/2} \qquad (2)$$

The term "hildebrands" has been adopted for the solubility parameter units. As an illustration, Table 1 shows several solvents in order of increasing Hildebrand parameter. The Hildebrand parameter is given in a value conforming to standard international units (SI units). The SI unit of the Hildebrand parameter is given as $\delta/MPa^{1/2}$.

Table 1

| Hildebrand Solubility Parameters of Selected Solvents | |
|---|---|
| **Solvent** | **$\delta$(SI)** |
| n-Pentane | 14.4 |
| Ethyl acetate | 18.2 |
| Acetone | 19.7 |
| Ethyl alcohol | 26.2 |
| Methyl alcohol | 29.7 |
| Glycerol | 36.2 |
| Water | 48.0 |

Hildebrand parameters for a polymer can be determined experimentally by observing the degree of swelling of the polymer in a "spectrum" of liquids having a range of Hildebrand parameters. For example, a slightly crosslinked polymer is exposed to a series of liquids. The polymer is swollen but does not dissolve because of the crosslinks. The extent of swelling is plotted against the Hildebrand parameters of the liquids. Experimentally, some scatter is observed in the data, but the general trend is usually clear and an appropriate single value or range of Hildeband values can be obtained from the position of the maximum of the curve of swelling versus solvent. In this way, Hildebrand solubility parameters for a wide range of polymers have been reported in the literature. Such polymers include polyacrylates, polyamides, polyesters, polyethers (including polyethylene oxide and polypropylene oxide), ethylene propylene copolymers and terpolymers, polyisobutylene, polypropylene, silicones, polystyrene, polyvinyl acetate, and polyvinylpyrrolidone. The swelling method and other methods of determining solubility parameters for polymers is given in Chapter 14 of the Handbook of Solubility Parameters and Other Cohesion Parameters by Allan Barton, CRC Press (1991) and references cited therein, the full disclosures of which are incorporated by reference. Also, software is now available to make such calculations easier, for example, using the Sybyl Molecular Modeling Program 6 (available from Tripos Associates) to calculate Hildebrand solubility parameters using a structured base model. Other discussion of Hildebrand solubility parameters is provided for example in Guenin et al. (U.S. Patent No. 6,036,964) especially at columns 5 and 6, the disclosure of which is hereby incorporated by reference.

Swellable Polymers

The shell of the capsule contains a first polymer that functions as a structural polymer. The capsules further contain a second polymer that exhibits greater swelling in water than the first polymer. In various embodiments, the greater swelling in water is expressed as a higher value of the Hildebrand solubility parameter. In various aspects, the more soluble second polymer has a solubility parameter of at least 0.5 hildebrands greater than that of the first polymer. In other embodiments, the more swellable second polymer has a solubility parameter of at least 1 hildebrand greater than that of the first polymer.

Non-limiting examples of polymers that may serve as the first polymer include polylactic acid, polyglycolic acid, polylactic-co-glycolic acid, polycaprolactone, polyphosphoester, polyvinyl acetate, polystyrene, polyglucosamine, gelatin, and gum arabic. All of these materials have Hildebrand solubility parameters that are reported in the literature or can be assessed using experimental methods described herein.

Non-limiting examples of swellable materials that may serve as the second polymer include polyethylene oxide, polyacrylic acid, polyvinylpyrrolidone, polyvinyl alcohol, polyvinyl methyl ether-co-maleic acid, hyaluronic acid, and polysaccharides. Examples of the polysaccharides include gum arabic, alginate, carboxymethyl cellulose, hydroxypropylmethyl cellulose, hydroxyethyl cellulose, starch, and the like.

The first polymer can be selected from the possibilities for the second polymer and vice versa as long as the selection for the second polymer has the higher degree of swelling in water.

As the second polymer in the capsules absorbs water and swell to a greater degree than the first polymer, in various embodiments, they function as a kind of "bandage" to in effect seal the wall material off from passage of water through the wall from the outside to the core or active materials from the core to the outside. As noted above, they function as a bandage by absorbing water and swelling, thereby decreasing the mean free space in the shell wall and increasing the length of the tortuous pathway for materials crossing the boundary of the shell wall.

In various embodiments, the second polymer is crosslinked so that, even though it swells with water to a greater extent than the at least one first polymer, it will not dissolve in water. In various embodiments, crosslinking is carried out by conventional means, including irradiation crosslinking and chemical crosslinking. Radiation crosslinking includes crosslinking by electron beams, ultraviolet, γ-irradiation, x-rays, and other means. Chemical crosslinking is carried out by exposing the polymers to the crosslinking composition or molecule that contains a plurality of functional groups that are reactive with functional groups on the polymer. To illustrate, polymers having hydroxyl functional groups can be crosslinked by crosslinking compositions containing a plurality of hydroxyl reactive groups such as carboxyls, aldehydes, methylol groups, or isocyanates. For example, glutaraldehyde can be used to crosslink the second polymer.

Capsules

Capsules containing the first and second polymer tend to be more impermeable to water. As a result, encapsulated active materials are more stable in aqueous environments because the passage of water through the walls is inhibited by the presence of the second "bandage" polymer. The capsules are thus a kind of "extended" or "delayed" release composition, wherein the active materials are released over a prolonged period compared to capsules made by conventional means. The extent of the delayed or prolonged release of the actives from the capsules can be controlled or regulated by the nature and level of the at least one second polymer in the capsules as described herein. Alternatively, extended release compositions containing active ingredients can be formulated by combining conventional capsules showing a relatively fast release of actives with other capsules made by the methods described herein. Thus, conventional capsules can be provided for faster or instant release of the active materials, while the "bandaged" capsules described herein can be provided for a slower or more delayed release of the actives. Capsules described herein can be used in a wide variety of applications. Non-limiting examples include oral care such as aqueous oral care compositions, toothpaste and mouthwashes; personal care, such as antiperspirants and deodorants; shampoo; conditioner; body washes; bar soaps; shave creams; cosmetics; lotions; and home care such as hard surface cleaner, dishwashing liquids, light beauty liquids, auto dish, laundry detergents, heavy duty detergents, and fabric softeners.

In these and other applications, a variety of active materials can be encapsulated to aid in formulating the compositions. Non-limiting examples of actives include flavors and colorants, antimicrobial agents, reactive materials such as peroxides, and chemically sensitive materials such as, for example, enzymes. Such active materials may be incorporated into capsules by conventional means, using the first and second polymers described herein. Non-limiting examples of active ingredients included in the core of the capsules include edible oils, paraffin oils, silicone oils, proteins, keratin, collagen, casein, lecithin, sorbitol, antioxidants, phenol derivatives, antimicrobial agents, anti-inflammatory substances, caries-inhibiting substances, vitamins, enzymes, plant extracts, preservatives, pH regulators, sweeteners, flavorings, and perfumes. Flavorings and perfumes include without limitation essential oils and extracts, tinctures and balsams, for example, anise seed oil, basil oil, camphor oil, citronella oil, eucalyptus oil, chamomile oil, mint oil, lime oil, clove oil, peppermint oil, sage oil, thyme oil, vanilla extract, cinnamon bark oil, and the like. Additional actives include substances having a cooling or refreshing effect in the mouth, throat, or nasal cavity. Non-limiting examples include menthol, eucalyptol, thymol, methyl salicylate, and the like.

Capsules containing the active ingredients can be formulated into a number of physical forms, including creams, gels, foams, dispersions, chewing gums, pastilles, and lozenges. In the oral care area, the capsules can be formulated into toothpastes, tooth creams, and tooth gels, without limitation.

Microencapsulation

Capsules containing active materials in the core and first and second polymers providing enhanced impermeability to water are made by conventional means that incorporate both the first and second polymer described herein. For example, coacervation can be carried out using either aqueous phase separation (oil in water encapsulation) or organic phase separation (water in oil encapsulation), depending on whether the core material is an oil or a polar material. To illustrate for an organic phase separation process, a polar core is dispersed into an oily or non-polar continuous medium. The wall material is then dissolved in the continuous medium. The wall material includes the at least one first polymer above and may further contain the second polymer. Alternatively, the second polymer is provided in the polar core. After heating to an elevated temperature and holding it for a suitable time, for example one hour, the system is allowed to cool rapidly. Upon cooling the capsules form.

Carriers

In various embodiments, the capsules are formulated together with an acceptable carrier to provide compositions useful in a variety of applications. Depending on the form of the composition, the acceptable carrier can be a liquid carrier, a powder carrier, a dissolvable solid carrier, a gum base, a film forming polymer or polymers, and so on.

In the oral care area, the compositions are said to contain an orally acceptable carrier in addition to the capsules. As used herein, the "carrier" refers to components of the individual oral compositions in which the capsules are formulated as an active ingredient. In various embodiments, the carrier embraces all of the components of the oral composition except for those in the capsules. In other aspects, the term refers to components such as inactive ingredients, carriers, vehicles, and the like, that are commonly understood to persons of skill in the art to function as a carrier, filler, or other relatively inert ingredient. In other words, the term carrier is used in different ways depending on context. Depending on the context, the oral compositions may comprise other components in addition to the capsules and the carrier. However, in all contexts, the components of the oral compositions of the invention can be divided into carrier components and capsules.

To illustrate in a non-limiting example for the case of toothpastes, the carrier can be said to be the water/humectant system that provides a large fraction by weight of the composition. Alternatively, the carrier component of a toothpaste composition may be considered as the water, humectant, and other functional components other than the capsules. Whatever the context, the person of skill in the art recognizes that the toothpaste composition contains both capsules and an orally acceptable carrier.

To illustrate further, in a mouth rinse, the carrier is generally considered to be the water/alcohol liquid component in which the active ingredients are dissolved or dispersed. In a dissolvable lozenge, the carrier is generally understood to comprise the solid matrix material that dissolves slowly in the mouth to the oral surfaces in the mouth. In chewing gums, the carrier comprises a gum base, while in an edible strip, the carrier comprises one or more film forming polymers.

In all of the above examples, the oral composition, in whatever form, includes the capsules, a suitable carrier in an appropriate form, and other actives or functional materials needed to provide the oral compositions with desired properties. Additional active materials and functional materials are described below.

In addition to the capsules, a number of active ingredients and functional materials are included in various compositions of the invention. Such materials include, without limitation, abrasives, humectants, surfactants, anticalculus agents, thickeners, viscosity modifiers, anticaries agents, flavorants, colorants, additional antibacterial agents, antioxidants, anti-inflammation components, and so on. They are added to the pastes, rinses, gums, lozenges, strips, and other forms of the oral compositions of the invention according to known methods.

EXAMPLES

The following non-limiting example can be prepared by mixing of the ingredients. The amounts are based weight percent of the total composition.

| Material | Weight % |
| --- | --- |
| Water | q.s. |
| Sorbitol | 58 |
| Polyethylene glycol (600 MW) | 3 |
| Cocamidopropyl Betaine | 1.25 |
| Sodium Lauryl Sulfate | 1.5 |
| Carboxymethylcellulose Gum | 0.65 |
| Capsules | 2 |
| Na Saccharin | 0.3 |
| High Cleaning Silica | 20 |
| Thickening Silica | 4.25 |
| Tetrasodium Pyrophosphate | 0.5 |

(continued)

| Material | Weight % |
|---|---|
| Sodium Fluoride | 0.243 |
| Blue Color | 0.1 |
| Flavor | 1.2 |
| Total | 100 |

**Claims**

1. A capsule comprising a core and a shell surrounding and enclosing the core, the shell comprising

   a) a first polymer having a first Hildebrand solubility parameter, and
   b) a second polymer having Hildebrand solubility parameter higher than the first water solubility parameter,

   and wherein

   i) the second polymer is crosslinked, and
   ii) the second polymer is present in an amount of from 0.01% to less than 50% of the total weight of the first and second polymers in the capsule,

   wherein the first polymer is selected from polylactic acid, polyglycolic acid, polylactic-co-glycolic acid, polycaprolactone, polyphosphoester, polyvinyl acetate, polystyrene, polyglucosamine, gelatin and gum arabic, and wherein the second polymer is selected from polyethylene oxide, polyacrylic acid, polyvinylpyrrolidone, polyvinyl alcohol, polyvinylmethylether-co-maleic acid, hyaluronic acid, alginate, carboxymethyl cellulose, hydroxypropylmethyl cellulose, hydroxyethyl cellulose and starch.

2. The capsule of claim 1, wherein the core comprises 1 to 40 parts by weight of the second polymer per 100 parts total of the first and second polymer.

3. The capsule of claim 1, wherein the solubility parameter of the second polymer is at least 0.5 Hildebrand units greater than the solubility parameter of the first polymer.

4. The capsule of claim 1, wherein the solubility parameter of the second polymer is at least 1 Hildebrand units greater than the solubility parameter of the first polymer.

5. The capsule of claim 1, further comprising an active material.

6. The capsule of claim 5, wherein the active material is an oral care ingredient.

7. An oral care composition comprising the capsule of claim 1, wherein the composition comprises an orally acceptable carrier.

8. A method of decreasing the water permeability of a capsule shell comprising forming a capsule from a mixture of:

   a) a first polymer having a first Hildebrand solubility parameter, and
   b) a second polymer having a Hildebrand solubility parameter higher than the first water solubility parameter,

   and wherein

   i) the second polymer is crosslinked, and
   ii) the second polymer is present in an amount of from 0.01% to less than 50% of the total weight of the first and second polymers in the capsule,

   wherein the first polymer is selected from polylactic acid, polyglycolic acid, polylactic-co-glycolic acid, polycaprolactone, polyphosphoester, polyvinyl acetate, polystyrene, polyglucosamine, gelatin and gum arabic, and wherein the second polymer is selected from polyethylene oxide, polyacrylic acid, polyvinylpyrrolidone, polyvinyl alcohol, polyvinylmethylether-co-maleic acid, hyaluronic acid, alginate, carboxymethyl cellulose, hydroxypropylmethyl cellulose, hydroxyethyl cellulose and starch.

9. The method of claim 8, wherein the core comprises 1 to 40 parts by weight of the second polymer per 100 parts total of the first and second polymer.

10. The method of claim 8, wherein the solubility parameter of the second polymer is at least 0.5 Hildebrand units greater than the solubility parameter of the first polymer.

11. The method of claim 8, wherein the solubility parameter of the second polymer is at least 1 Hildebrand units greater than the solubility parameter of the first polymer.

## Patentansprüche

1. Kapsel, umfassend einen Kern und eine Hülle, die den Kern umgibt und einschließt, wobei die Hülle umfasst

   a) ein erstes Polymer mit einem ersten Hildebrand-Löslichkeitsparameter, und
   b) ein zweites Polymer mit einem Hildebrand-Löslichkeitsparameter, der höher ist als der erste Wasserlöslichkeitsparameter,

   und wobei

   i) das zweite Polymer quervernetzt ist, und
   ii) das zweite Polymer in einer Menge von 0,01% bis weniger als 50% des Gesamtgewichts des ersten und zweiten Polymers in der Kapsel vorliegt,

   wobei das erste Polymer ausgewählt ist aus, Polylactid, Polyglycolid, Polylactid-coglycolid, Polycaprolacton, Polyphosphoester, Polyvinylacetat, Polystyrol, Polyglucosamin, Gelatine und Gummiarabikum, und wobei das zweite Polymer ausgewählt ist aus Polyethylenoxid, Polyacrylsäure, Polyvinylpyrrolidon, Polyvinylalkohol, Polyvinylmethylether-co-maleinsäure, Hyaluronsäure, Alginat, Carboxymethylcellulose, Hydroxypropylmethylcellulose, Hydroxyethylcellulose und Stärke.

2. Die Kapsel nach Anspruch 1, wobei der Kern 1 bis 40 Gewichtsteile des zweiten Polymers pro 100 Teile des ersten und zweiten Polymers insgesamt umfasst.

3. Die Kapsel nach Anspruch 1, wobei der Löslichkeitsparameter des zweiten Polymers mindestens 0,5 Hildebrand-Einheiten größer ist als der Löslichkeitsparameter des ersten Polymers.

4. Die Kapsel nach Anspruch 1, wobei der Löslichkeitsparameter des zweiten Polymers mindestens 1 Hildebrand-Einheiten größer ist als der Löslichkeitsparameter des ersten Polymers.

5. Die Kapsel nach Anspruch 1, weiterhin umfassend ein aktives Material.

6. Die Kapsel nach Anspruch 5, wobei das aktive Material ein Mundpflegeinhaltsstoff ist.

7. Die Mundpflegezusammensetzung umfassend die Kapsel nach Anspruch 1, wobei die Zusammensetzung einen oralverträglichen Träger umfasst.

8. Verfahren zum Verringern der Wasserpermeabilität einer Kapselhülle umfassend das Bilden einer Kapsel aus einer Mischung von:

   a) einem ersten Polymer mit einem ersten Hildebrand-Löslichkeitsparameter, und
   b) einem zweiten Polymer mit einem Hildebrand-Löslichkeitsparameter, der höher ist als der erste Wasserlöslichkeitsparameter,

und wobei

i) das zweite Polymer quervernetzt ist, und
ii) das zweite Polymer in einer Menge von 0,01% bis weniger als 50% des Gesamtgewichts des ersten und zweiten Polymers in der Kapsel vorliegt,

wobei das erste Polymer ausgewählt ist aus Polylactid, Polyglycolid, Polylactid-coglycolid, Polycaprolacton, Polyphosphoester, Polyvinylacetat, Polystyrol, Polyglucosamin, Gelatine und Gummiarabikum, und wobei das zweite Polymer ausgewählt ist aus Polyethylenoxid, Polyacrylsäure, Polyvinylpyrrolidon, Polyvinylalkohol, Polyvinylmethylether-co-maleinsäure, Hyaluronsäure, Alginat, Carboxymethylcellulose, Hydroxypropylmethylcellulose, Hydroxyethylcellulose und Stärke.

9. Das Verfahren nach Anspruch 8, wobei der Kern 1 bis 40 Gewichtsteile des zweiten Polymers pro 100 Teile des ersten und zweiten Polymers insgesamt aufweist.

10. Das Verfahren nach Anspruch 8, wobei der Löslichkeitsparameter des zweiten Polymers mindestens 0,5 Hildebrand-Einheiten größer ist als der Löslichkeitsparameter des ersten Polymers.

11. Das Verfahren nach Anspruch 8, wobei der Löslichkeitsparameter des zweiten Polymers mindestens 1 Hildebrand-Einheiten größer ist als der Löslichkeitsparameter des ersten Polymers.

## Revendications

1. Capsule comprenant un noyau et une enveloppe entourant et enfermant le noyau, l'enveloppe comprenant :

a) un premier polymère ayant un premier paramètre de solubilité d'Hildebrand, et
b) un second polymère ayant un paramètre de solubilité d'Hildebrand supérieur au premier paramètre de solubilité dans l'eau,

et dans laquelle

i) le second polymère est réticulé, et
ii) le second polymère est présent en une quantité allant de 0,01 % à moins de 50 % du poids total des premier et second polymères dans la capsule.

dans laquelle le premier polymère est choisi parmi l'acide polylactique, l'acide polyglycolique, l'acide polylactique-co-glycolique, la polycaprolactone, un polyphosphoester, le poly(acétate de vinyle), le polystyrène, la polyglucosamine, la gélatine et la gomme arabique, et dans laquelle le second polymère est choisi parmi le poly(éthylène oxyde), l'acide polyacrylique, la polyvinylpyrrolidone, le poly(alcool de vinyle), le polyvinylméthyléther-co-acide maléique, l'acide hyaluronique, l'alginate, la carboxyméthylcellulose, l'hydroxypropylméthylcellulose, l'hydroxyéthylcellulose et l'amidon.

2. Capsule selon la revendication 1, dans laquelle le noyau comprend 1 à 40 parties en poids du deuxième polymère pour 100 parties au total des premier et deuxième polymères.

3. Capsule selon la revendication 1, dans laquelle le paramètre de solubilité du second polymère est supérieur d'au moins 0,5 unité Hildebrand au paramètre de solubilité du premier polymère.

4. Capsule selon la revendication 1, dans laquelle le paramètre de solubilité du second polymère est supérieur d'au moins 1 unité Hildebrand au paramètre de solubilité du premier polymère.

5. Capsule selon la revendication 1, comprenant en outre une matière active.

6. Capsule selon la revendication 5, dans laquelle la matière active est un ingrédient de soin buccal.

7. Composition de soin buccal comprenant la capsule selon la revendication 1, dans laquelle la composition comprend un support acceptable par voie orale.

**8.** Procédé pour diminuer la perméabilité à l'eau d'une enveloppe de capsule comprenant la formation d'une capsule à partir d'un mélange de :

a) un premier polymère ayant un premier paramètre de solubilité d'Hildebrand, et
b) un second polymère ayant un paramètre de solubilité d'Hildebrand supérieur au premier paramètre de solubilité dans l'eau,

et dans lequel

i) le second polymère est réticulé, et
ii) le second polymère est présent en une quantité allant de 0,01 % à moins de 50 % du poids total des premier et second polymères dans la capsule,

dans lequel le premier polymère est choisi parmi l'acide polylactique, l'acide polyglycolique, l'acide polylactique-co-glycolique, la polycaprolactone, un polyphosphoester, le poly(acétate de vinyle), le polystyrène, la polyglucosamine, la gélatine et la gomme arabique, et dans lequel le second polymère est choisi parmi le poly(éthylène oxyde), l'acide polyacrylique, la polyvinylpyrrolidone, le poly(alcool de vinyle), le polyvinylméthyléther-co-acide maléique, l'acide hyaluronique, l'alginate, la carboxyméthylcellulose, l'hydroxypropylméthylcellulose, l'hydroxyéthylcellulose et l'amidon.

**9.** Procédé selon la revendication 8, dans lequel le noyau comprend 1 à 40 parties en poids du deuxième polymère pour 100 parties au total des premier et deuxième polymères.

**10.** Procédé selon la revendication 8, dans lequel le paramètre de solubilité du second polymère est supérieur d'au moins 0,5 unité Hildebrand au paramètre de solubilité du premier polymère.

**11.** Procédé selon la revendication 8, dans lequel le paramètre de solubilité du second polymère est supérieur d'au moins 1 unité Hildebrand au paramètre de solubilité du premier polymère.

## REFERENCES CITED IN THE DESCRIPTION

**Patent documents cited in the description**

• US 6036964 A, Guenin

**Non-patent literature cited in the description**

• **ALLAN BARTON.** Handbook of Solubility Parameters and Other Cohesion Parameters. CRC Press, 1991